# EUROPEAN PATENT SPECIFICATION

(11) **EP 1 443 984 B1**
(45) Date of publication and mention of the grant of the patent: **30.03.2005**
(21) Application number: 02789643.0
(22) Date of filing: 13.11.2002
(51) Int. Cl.: A61L 33/00

(54) **SPRAY DRY PROCESS FOR APPLYING ANTICOAGULANT ON A SYRINGE BARREL**
SPÜHTROCKNUNGS-VERFAHREN ZUM AUFBRINGEN VON GERINNUNGSHEMMERN IN EINEN SPRITZENKOLBEN
PROCEDE DE DESSICCATION PAR ATOMISATION PERMETTANT D'APPLIQUER UN ANTICOAGULANT SUR UN CYLINDRE DE SERINGUE

(30) Priority: 13.11.2001 US 350613 P
(43) Date of publication of application: 11.08.2004
(73) Proprietor: Becton Dickinson and Company, Franklin Lakes, New Jersey 07417 (US)
(72) Inventor: BARKELL, Paul, Plymouth, Devon PL7 4EF (GB); CHURCH, Stephen, Plymouth, Devon PL7 4EF (GB)
(74) Representative: Weber, Thomas Dr.
(86) International application number: PCT/US2002/036545
(87) International publication number: WO 2003/041759

(56) References cited:
- EP-A- 0 609 794
- US-A- 4 529 614
- US-A- 4 808 449
- PATENT ABSTRACTS OF JAPAN vol. 1995, no. 09, 31 October 1995 (1995-10-31) & JP 07 149544 A (SHIBUYA KOGYO CO LTD), 13 June 1995 (1995-06-13)
- PATENT ABSTRACTS OF JAPAN vol. 1996, no. 09, 30 September 1996 (1996-09-30) & JP 08 112560 A (TOKYO COPAL KAGAKU KK;MEIKI JUSHI KOGYO KK), 7 May 1996 (1996-05-07)

## Description

### 1. Field of Invention

The present invention is directed to a method for applying anticoagulants to a substrate. More particularly, the present invention is directed to a spray dry process for applying anticoagulants onto a syringe barrel.

### 2. Description of Related Art

Syringes are often used to take blood samples from patients. In some cases it is desirable for the blood sample not to clot. In such cases an anticoagulant is typically added to the syringe to prevent the clotting of the blood sample. The addition of an anticoagulant is often performed at the manufacturing step as compared to a medical technician adding a quantity of anticoagulant prior to the taking of a blood sample.

An anticoagulant has been added into an interior portion of a syringe as either a liquid anticoagulant or a lyophilized anticoagulant. The addition of liquid anticoagulant or lyophilized anticoagulant, however, has a number of problems. Liquid anticoagulants should be retained within the syringe barrel and prevented from escaping therefrom. This often presents problems with the user of such a syringe because additional care must be taken to prevent the escape or leakage of the anticoagulant. For instance, occasionally a liquid anticoagulant may escape due to a dislodged or missing cap that seals an open end of the syringe tube. Furthermore, many anticoagulants in their liquid form, such as liquid heparin, are unstable and may be further degraded by a number of subsequent manufacturing processes, such as gamma sterilization.

Attempts have been made to avoid the problems associated with the potential leakage of a liquid anticoagulant from a syringe. Syringe barrels have been immersed in an anticoagulant solution or coated with an anticoagulant solution. The syringe barrel is then dried to provide a coating of anticoagulant in particulate form. The drying step, however, further complicates such a procedure. Ambient drying often takes many hours, adding to the cost of manufacturing an anticoagulant-coated syringe. Thermal heat has been applied by the use of ovens or by the flow of hot gas, such as air at about 90°C or greater, to reduce the drying time, but such techniques also add additional processing steps. Forced ambient air drying has been used where an anticoagulant has been dissolved in a volatile organic solvent, but such drying is apparently not as effective for aqueous solutions of anticoagulants as compared to forced hot air drying. In all of these cases, however, the drying technique is either too time consuming or adds complicating steps, such as the use of an organic solvent.

To avoid the problems associated with liquid anticoagulants, anticoagulants have been chemically bonded, i.e., covalent or ionic bonding, to an interior portion of a syringe or to a linking agent which is itself bonded to a portion of the syringe. Such a syringe is then often rinsed and dried to remove unreacted chemicals. Such a syringe, however, must often have its substrate pretreated so that it is receptive to the bonding of the anticoagulant or the linking agent. Such techniques add time-consuming and often costly steps by first pretreating a syringe surface, coating the surface with a chemical linking agent followed by a second coating with an anticoagulant, setting appropriate reaction times and conditions, and removing unreacted reactants or undesirable reaction products.

To avoid the application of a liquid anticoagulant, lyophilized anticoagulant has been used as a deposit on an interior portion of a syringe barrel. The lyophilized anticoagulant, however, must be protected from moisture to avoid dissolving of the lyophilized anticoagulant. Thus, lyophilized anticoagulant is often stored in packaging that ensures the presence of an adequate moisture barrier. Furthermore, the application of lyophilized antocoagulant such as heparin into syringes is a difficult process. The lyophilized anticoagulant is often blow into the syringes as what is commonly called "puffs" of anticoagulant. The application of the "puffs" to a syringe barrel is often performed manually and prone to error. For instance, on occasion a syringe may contain only a partial "puff" or no "puff" at all due to operator oversight The blood sample contained in such a syringe will typically clot, causing analytical problems and delays.

There is a need for an improved method of applying anticoagulants to a substrate without the above-described disadvantages. In particular, there is a need for a method of applying anticoagulants to a syringe barrel which ensures proper application and securement of the anticoagulant within the syringe without costly affixation steps and equipment.

### 3. Summary of the Invention

The process of the present invention coats a portion of an interior surface of a syringe barrel with an anticoagulant using a spray and dry process. The anticoagulant is deposited to physically coat the portion of the interior surface as compared to chemically bonding the anticoagulant thereat. Concentrated aqueous anticoagulant solutions are advantageously used with the practice of the present invention.

In one aspect of the present invention a method for coating a substrate with an anticoagulant is provided. The inventive method includes providing a concentrated aqueous anticoagulant solution, providing a flow of pressurized air and providing a spray nozzle having a first chamber in fluid communication with the flow of pressurized air and having a second chamber in fluid communication with the anticoagulant solution. The anticoagulant solution is atomized into fine mist droplets as the anticoagulant solution exits the second chamber of the spray nozzle. The pressurized air flowing through the first chamber of the spray nozzle, in part, directs the droplets towards a surface of a substrate to coat the surface with the anticoagulant solution.

In another aspect of the present invention a method for coating an interior portion of a syringe with an anticoagulant includes providing a concentrated aqueous anticoagulant solution, providing a flow of pressurized air, and providing at least two spray nozzles each having a first chamber in fluid communication with the flow of pressurized air and each having a second chamber in fluid communication with the anticoagulant solution. From about 1 to about 20 microliters of the anticoagulant solution is atomized into fine mist droplets as the anticoagulant solution exits the second chamber of one of the spray nozzles. These droplets are directed into the flow of pressurized air exiting the one spray nozzle to, in part, direct the droplets towards an interior portion of a syringe to physically coat the portion with the anticoagulant solution. Moreover, the other spray nozzle is then used to atomize from about 1 to about 20 microliters of the anticoagulant solution into fine mist droplets as the anticoagulant solution exits the second chamber of the other of the spray nozzle. This second spray is directed into the flow of pressurized air exiting this spray nozzle to, in part, direct the droplets towards the portion of the syringe to physically coat the portion with the anticoagulant solution. The droplets of anticoagulant solution on the surface of the substrate are dried by forcing warm air over the surface to evaporate water from the anticoagulant solution to leave a physical coating of solid anticoagulant thereat.

A syringe for collecting a blood sample is also provided. The syringe includes an interior portion having a physical coating of air-dried anticoagulant, such as calcium balanced lithium heparin thereat. The coating being deposited by atomizing from 1 to 20 microliters of concentrated aqueous calcium balanced lithium heparin solution.

Spray coating operation eliminates the possibility of heparin loss in liquid systems as the heparin is coated onto the syringe barrel interior surface and remains fixed thereon. The dried heparin is also more stable when exposed to subsequent processes such as gamma irradiation.

### 4. Description of the Drawings

Figure 1 is a schematic depiction of the method of the present invention for depositing an anticoagulant onto a surface of a substrate.

Figure 2 is a schematic depiction of the anticoagulant delivery system of the present invention including a spray nozzle for atomizing the anticoagulant.

Figure 3 is a cross section view of a spray nozzle of the delivery system of Figure 2 taken along the 3-3 axis.

Figure 4 is a depiction of a blood syringe having an interior portion coated with the anticoagulant.

Figure 5 is a cross sectional view of the syringe of Figure 4 showing a layer of anticoagulant on an interior portion of the syringe taken along the 5-5 axis.

### 5. Detailed Description

The present invention includes a method for applying an anticoagulant onto substrates. The present invention is applicable to a wide variety of substrates including, for example, glass, metal or resin materials. Resin materials include, but are not limited to, polyethylene, acrylonitrile-butadiene-stryene terpolymer, polystryrene, polyester, for instance, polyethylene terephthalate and nylon as well as other substrate materials where it is desirable to impart a coating of an anticoagulant thereon.

Useful substrates include interior portions of medical containers, such as, tubes or syringes. Such tubes or syringes are often configured to hold only milliliters of a blood sample, such as one to five milliliters. The present invention is especially useful in coating interior portions of these milliliter-sized containers because of the controlled delivery of small quantities of an anticoagulant. For example, a spray of only 12 microliters of an anticoagulant may be used with the practice of the present invention to coat one to five milliliter-sized blood syringes.

To aid the flow of blood within these milliliter-sized containers, it is often desirable to have a hydrophobic substrate or impart hydrophobicity thereon. In one aspect of the present invention the interior portion of these milliliter-sized containers are precoated with silicone oil to impart hydrophobicity. The silicone oil is an organopolysilane that is not chemically reactive with the substrate or the anticoagulant. Suitable organopolysiloxanes are commercially available, for example "DC193" silicone supplied by Dow Corning.

The anticoagulant is sprayed or atomized onto the surface of the substrate. Such atomization imparts coverage of fine mist of the anticoagulant solution onto a substrate. Such a fine mist aids in the even coverage of the anticoagulant over the substrate. Atomization also helps in the subsequent drying of the anticoagulant solution, which typically contains deionized water, because the fine mist results in small droplets of anticoagulant deposited on the substrate which increases the surface area of such small droplets, thereby easing the drying of the deionized water. Moreover, a hydrophobic substrate or a substrate with hydrophobicity imparted thereto will also aid in dispersion and drying of the anticoagulant by repelling the water from the solution forming smaller droplets.

Anticoagulants useful with the practice of the present invention include those anticoagulants that can be provided in an aqueous solution. Examples of such anticoagulants include, but are not limited to, lithium heparin, ammonium heparin, sodium heparin, ethylene diamine tetraacetic acid (EDTA), acid citrate dextrose (ACD), sodium citrate, citrate phosphate dextrose (CPD), sodium fluoride, sodium oxalate, potassium oxalate, lithium oxalate, sodium iodoacetate and lithium iodoacetate. A useful anticoagulant includes calcium balanced lithium heparin. Moreover, concentrated aqueous solutions of an anticoagulant are useful with the practice of the present invention. For example, a calcium balanced lithium heparin solution having a heparin concentration from about 3000 to about 7,500 IU per milliliter (USP) is useful with the practice of the present invention. Such a heparin solution may also contain from about 12.0 to about 13.2 mmol/L of calcium.

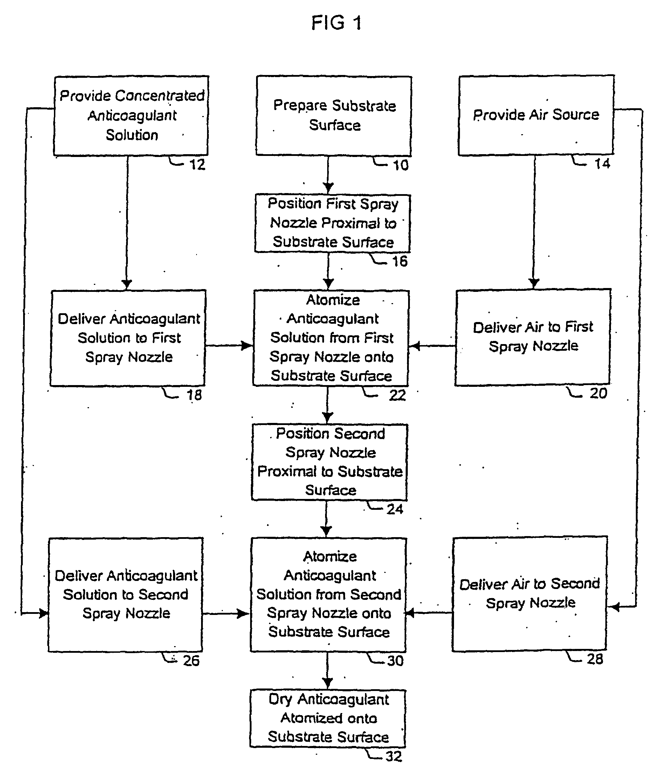

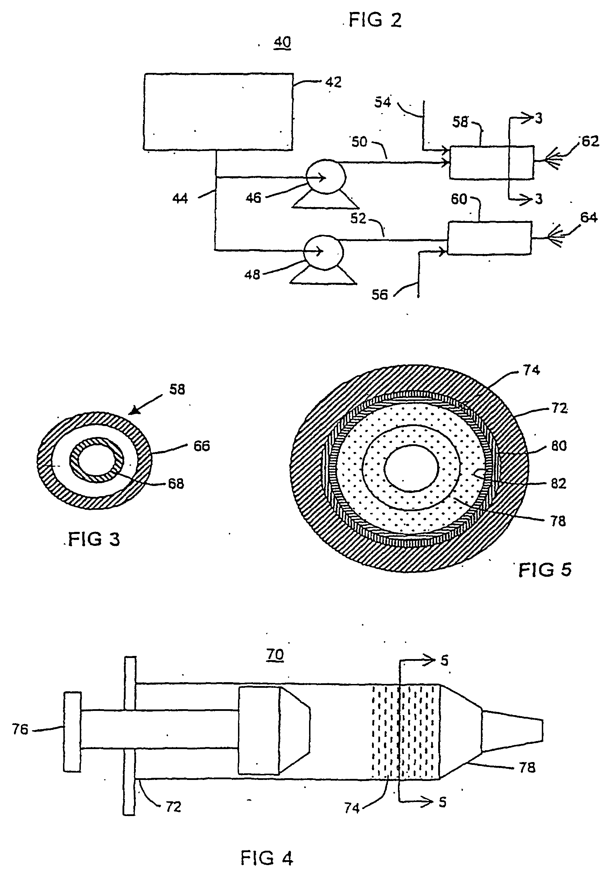

The method of the present invention for coating a surface of a substrate with a concentrated solution of anticoagulant is depicted in Figures 1 and 2. Figure 1 is a schematic depiction of the method of the present invention. Figure 2 is a schematic illustration of the coating assembly 40 used with the inventive method.

At step 10 of Figure 1, the substrate surface is prepared. Preparation may include cleaning of the surface, if necessary. Moreover, as discussed above, hydrophobic substrates or substrate surfaces having hydrophobicity are useful with the present invention. Coating the surface of the substrate with silicone oil, is a useful means for providing hydrophobicity to substrate surfaces.

At step 12, a concentrated anticoagulant solution is provided in reservoir 42. Desirably, the concentrated anticoagulant solution is a concentrated aqueous solution of the anticoagulant. More desirably, the solution is a calcium balanced lithium heparin aqueous solution. A useful concentration includes, but not limited to, from about 3,000 to about 10,000 IU per milliliter (USP) of heparin, more desirably from about 6,500 to about 7,500 IU per milliliter (USP) of heparin.

At step 14, an air source 54 is provided. The air source is typically pressurized to facilitate the delivery of air to a spray nozzle. Low pressure air, i.e., 62 kilopascals gauge or 9 psig, may be suitably used with the practice of the present invention.

At step 16, a first spray nozzle 58 is positioned proximal to the substrate which is to be coated by the present invention. When the substrate includes an interior portion of a syringe, the first spray nozzle 58 may be suitably positioned within an interior portion of the syringe.

At steps 18 and 20, anticoagulant and air are directed to the first spray nozzle 58. The first spray nozzle 58 includes a tube 66 and a cannula 68. Cannula 68 is positioned within tube 66. Cannula 68 is in fluid communication with the liquid anticoagulant so that the anticoagulant may travel through its interior bore or cavity. Tube 66 is in fluid communication with the air source 54 so that pressurized air may travel through its interior bore or cavity. The inner diameter of the tube 66 is larger than the outer diameter of the cannula 68 to permit the passage of air through interior portions of tube 66. As a nonlimiting example, tube 58 may be a 4-millimeter diameter outer tube and cannula 68 may be a 21G cannula (about 0.8 millimeter).

As depicted in Figure 2, pump 46 delivers the liquid anticoagulant from reservoir 42 to the first spray nozzle 58 via lines 44 and 50. Desirably, the pump 46 is a positive displacement metered syringe pump.

At step 22 the solution of anticoagulant is atomized onto the substrate surface. The atomization is achieved by pressurizing the anticoagulant solution through a small bore, such as the cannula 68. The pressurized air not only aids in the atomization of the anticoagulant, but also assists in controlling the flow and direction of the atomized anticoagulant 62. The amount of anticoagulant atomized and deposited on the substrate may vary. For example, the range of heparin often required to inhibit clotting is very wide, i.e., 3 to 100 IU/ml of blood. Desirably, an interior portion of a bolls syringe having a nominal size from about 1 cc to about 5 cc needs only from about 1 microliter to about 20 microliters of concentrated heparin physically deposited to coat the interior portion that may contact blood. More desirably, from about 10 microliters to about 14 microliters of concentrated heparin may be physically deposited to coat the interior portion of a syringe.

After the first spray nozzle 58 has sprayed its metered quantity of anticoagulant, a second spray nozzle 60 is positioned proximal to the substrate surface. A second pump 48 and a second air source 56 deliver anticoagulant solution and pressurized air, respectively, to the second spray nozzle 60. The second spray nozzle forms atomized anticoagulant 64 for coating the substrate surface. Pumps 46 and 48, air sources 54 and 56, and spray nozzles 58 and 60 are similarly designed. In other words, duplicate and independent assemblies are used to spray the substrate. In such a manner the process of the present invention is well suited for the simultaneous coating of a plurality of surfaces, and the duplicate and independent assemblies and steps are advantageously used to provide all substrate surfaces with an anticoagulant coating even in the event of a failure or misalignment of one particular piece of equipment.

After the substrate has been coated with fine droplets of anticoagulant solution, warm air is used to dry the substrate at step 32. During the drying step water is evaporated from the fine mist droplets of anticoagulant solution leaving behind a physically deposited coating of solid anticoagulant on the substrate surface. The anticoagulant is physically deposited onto the substrate surface and is not chemically, i.e., ionicly or covalently, bonded thereat.

The drying step may be done in several intervals with different dryers to ensure adequate drying. For example, the substrate may witness four drying steps with each step being performed by an independent dryer. Warm air at about 40°C to about 60°C is then forced a low velocity, such as 5 to 15 meters per second, to dry the substrate. Desirably, the warm air is at about 50°C and is forced at a velocity of about 9 to 10 meters per second. When concentrated solutions of aqueous anticoagulants are used the drying time for each drying cycle is advantageously short, for example from about 5 to about 60 seconds. One reason for the reduced drying time is that concentrated aqueous solutions of anticoagulant advantageously contain less water that must be removed as compared to more diluted aqueous solutions of anticoagulants. Another reason for the reduced drying time is the deposition of the anticoagulant solution by atomization, which minimized droplet size of the anticoagulant. Moreover, a substrate surface having hydrophobic properties also assists in the formation of small droplets at substrate surface.

The application of the liquid anticoagulant in multiple passes ensures that the possibility of failure in the field is significantly reduced. This is particularly important where syringes are used to collect samples for blood gas analysis, as the time from collection to analysis may be crucial in the treatment of the patient. If the blood sample clots the analyzer will be adversely affected and require intervention delaying blood analysis and patient treatment.

One method to apply anticoagulant solution to multiple substrate surfaces, such as syringe barrels, is to use multiple robots carrying a series of spraying nozzles. The nozzles use low-pressure air to atomize the solution, which is metered to the nozzles using syringe type pumps. Syringe barrels are carried in a fixed pattern on pallets by an automated handling system. The robots are arranged in series and each robot and nozzle combination dispense a fraction of the total dispense required as the pallet indexes below the robot station. Once the pallet has completed the requisite number of operations the pallet then passes under a series of drying nozzles which gently blow warm air into the syringe barrels to remove residual moisture.

Figure 4 depicts a syringe 70 having an interior portion 74 of syringe barrel 72 coated with an anticoagulant. The syringe stopper and plunger rod assembly 76 need not be coated with anticoagulant. The area of anticoagulant coating may extend into the base and luer tip 78.

Figure 5 depicts is cross sectional view of syringe barrel 72 having coating of anticoagulant 82 applied at interior portion 74 by the above-described methods. As also shown in Figure 5, the interior portion 74 may also have a silicone oil coating 80 positioned between the interior portion 74 and the anticoagulant coating 82.

The process is not limited to syringes or heparin and can be applied to any medical container and any anticoagulant or coagulant enhancing product. The drying operation can also occur between dispense stations and alternative coating steps.

The invention may be further understood with reference to the following nonlimiting examples.

### EXAMPLE 1: High Concentration of Anticoagulant

Anticoagulant used was calcium balanced lithium heparin. The heparin is in a concentrated aqueous solution having from about 3000 to about 7,450 IU per milliliter (USP). Such a solution is commercially available from Celsus Laboratories, Inc. The heparin solution was sprayed onto inner surfaces of a syringe barrel.

The spray nozzle or dispenser system for delivering the heparin solution onto the inner surfaces of the syringe includes a larger outer bore and a smaller inner bore. The larger outer bore was designed as a tube from which pressurized air was dispensed. The inner bore was designed as a cannula from which the liquid heparin was dispensed. The size of dispenser was a 4-millimeter diameter outer tube and a 21G cannula (about 0.8 millimeter) inner tube. Pressurized air at approximately 62 kilopascals gauge or 9 psig was supplied through the outer tube. The liquid heparin was pumped through the inner tube and the liquid heparin and was atomized into droplet form.

Multiple spray nozzles or dispensers were used to process individual syringe barrels. A first spray nozzle was used to spray a first amount of the liquid heparin solution onto the inner surface of a syringe barrel. A second spray nozzle was then used to spray a second amount of heparin solution onto the same inner surface of the syringe barrel. A total of 12 microliters of lithium heparin was atomized into the syringe barrel with each of the two sprays having delivered 6 microliters.

A separate spray pump was used for delivering the heparin solution to each spray nozzle. The spray pumps were positive displacement metered syringe pumps. The syringe pumps for the first and the second spray nozzle worked independently of one and the other.

The first nozzle was positioned inside the interior diameter of the syringe prior to dispensing the spray. A first spray pump delivered 6 microliters of liquid heparin to the first spray nozzle. The liquid heparin was atomized onto the syringe barrel. The first nozzle was retracted from the syringe barrel and the second was then positioned inside the syringe barrel. The second spray pump delivered 6 microliters of liquid heparin solution to the second spray nozzle. The liquid heparin was atomized onto the interior portion of the syringe barrel.

After deposition the atomized heparin, the syringe barrel was subjected to a drying process. Warm air at approximately 50°C was forced into the syringe barrel for about 39 seconds. The air velocity was approximately 9 to 10 meters per second. The drying process was repeated for a total of fours times.

This process was used to coat different sized syringes as described below in Table 1.

**Table 1**

| **Coating Interior Portions of Different Sized Syringes** | | | | |
|---|---|---|---|---|
| Concentration of the Heparin Solution Used | Syringe Size | Dispense Volume | | Dispense Quantity |
| IU/ml | ml or cc | Microliters | ml | IU |
| 7000 | 3 | 12 | 0.012 | 84 |
| 7000 | 5 | 12 | 0.012 | 84 |
| 3750 | 1 | 12 | 0.012 | 45 |

Although illustrative embodiments of the present invention have been described herein with reference to the accompanying drawings, it is to be understood that the invention is not limited to those precise embodiments, and that various other changes and modifications may be effected therein by one skilled in the art without departing from the scope or spirit of the invention.

## Claims

1. A method for coating a substrate with an anticoagulant comprising:
providing a concentrated aqueous anticoagulant solution;
providing a flow of pressurized air;
providing a nozzle having a first chamber in fluid communication with said flow of pressurized air and having a second chamber in fluid communication with said anticoagulant solution;
atomizing said anticoagulant solution into fine mist droplets as said anticoagulant solution exits said second chamber; and
directing said droplets into said flow of pressurized air to direct said droplets towards a surface of a substrate to coat said surface with said anticoagulant solution.

2. The method of claim 1 further comprising drying said anticoagulant solution on said surface of said substrate by forcing warm air over said surface to evaporate water from said anticoagulant solution to leave a coating of solid anticoagulant thereat.

3. The method of claim 2 wherein said warm air is at least about 40°C; and
further wherein said warm air is no more than about 60°C.

4. The method of claim 2 wherein said warm air is forced over said surface from at least about 5 seconds; and
further wherein said warm air is forced over said surface from at no more than about 60 seconds.

5. The method of claim 2 wherein said warm air is forced over said surface at a velocity of at least about 5 meters per second; and
further wherein said warm air is forced over said surface at a velocity is no greater than about 15 meters per second.

6. The method of claim 1 wherein said anticoagulant solution includes ethylene diamine tetraacetic acid or heparin.

7. The method of claim 1 wherein said anticoagulant solution is a calcium balanced lithium heparin having a heparin concentration of at least about 3,000 IU per milliliter; and
further wherein said anticoagulant solution has a heparin concentration of no more than about 10,000 IU per milliliter.

8. The method of claim 1 wherein said anticoagulant solution is a calcium balanced lithium heparin having a heparin concentration of at least about 6,500 IU per milliliter; and
further wherein said anticoagulant solution has a heparin concentration of no more than about 7,500 IU per milliliter.

9. The method of claim 1 wherein said surface is an interior portion of a syringe.

10. The method of claim 9 wherein said atomizing includes atomizing at least about a 1 microliter of said anticoagulant solution; and
further wherein said atomizing includes atomizing no more than about a 20 microliters of said anticoagulant solution.

11. The method of claim 1 further comprising the step of providing hydrophobicity at said surface of said substrate prior atomizing said anticoagulant solution.

12. The step of claim 11 wherein said step includes coating said surface with a silicone oil.

13. A method for coating an interior portion of a syringe with an anticoagulant comprising:
providing a concentrated aqueous anticoagulant solution;
providing a flow of pressurized air;
providing at least two spray nozzles each having a first chamber in fluid communication with said flow of pressurized air and each having a second chamber in fluid communication with said anticoagulant solution;
atomizing from about 1 to about 20 microliters of said anticoagulant solution into fine must droplets as said anticoagulant solution exits said second chamber of one of said spray nozzles;
directing said droplets into said flow of pressurized air exiting said one spray nozzle to direct said droplets towards an interior portion of a syringe to coat said portion with said anticoagulant solution;
atomizing from about 1 to about 20 microliters of said anticoagulant solution into fine must droplets as said anticoagulant solution exits said second chamber of the other of said spray nozzles;
directing said droplets into said flow of pressurized air exiting said other spray nozzle to direct said droplets towards said portion of said syringe to coat said portion with said anticoagulant solution; and
drying said anticoagulant solution on said surface of said substrate to evaporate water from said anticoagulant solution to leave a physical coating of solid anticoagulant thereat.

14. The method of claim 13 wherein said drying further includes forcing warm air over said surface.

15. The method of claim 13 wherein said anticoagulant solution is a calcium balanced lithium heparin having a heparin concentration of at least about 3,000 IU per milliliter; and
further wherein said anticoagulant solution has a heparin concentration of no more than about 10,000 IU per milliliter.

16. The method of claim 13 wherein said anticoagulant solution is a calcium balanced lithium heparin having a heparin concentration of at least about 6,500 IU per milliliter; and
further wherein said anticoagulant solution has a heparin concentration of no more than about 7,500 IU per milliliter.

17. The method of claim 13 further comprising coating said surface with a silicone oil prior to atomizing said anticoagulant solution.

18. A syringe for collecting a blood sample comprising:
an interior portion having a physical coating of air-dried calcium balanced lithium heparin; said coating being deposited by atomizing from 1 to 20 microliters of a concentrated aqueous calcium balanced lithium heparin solution.

19. The syringe of claim 18 wherein said concentrated aqueous calcium balanced lithium heparin solution contains at least about 3,000 IU per milliliter of heparin; and
further wherein said concentrated aqueous calcium balanced lithium heparin solution contains no more than about 10,000 IU per milliliter heparin.

20. The syringe of claim 19 wherein said syringe has a nominal size from about 1 cc to about 5 cc.

21. The syringe of claim 18 wherein said concentrated aqueous calcium balanced lithium heparin solution contains at least about 6,500 IU per milliliter of heparin; and
further wherein said concentrated aqueous calcium balanced lithium heparin solution contains no more than about 7,500 IU per milliliter heparin.

22. The syringe of claim 21 wherein said syringe has a nominal size from about 1 cc to about 5 cc.

## Patentansprüche

1. Verfahren zur Beschichtung eines Substrats mit einem Antikoagulans, umfassend:
Bereitstellen einer konzentrierten wässrigen Antikoagulans-Lösung;
Bereitstellen eines Druckluftstroms;
Bereitstellen einer Düse mit einer ersten Kammer in Fluid-Verbindung mit dem Druckluftstrom und einer zweiten Kammer in Fluid-Verbindung mit der Antikoagulans-Lösung;
Zerstäuben der Antikoagulans-Lösung zu feinen Nebeltröpfchen, während die Antikoagulans-Lösung aus der zweiten Kammer austritt; und
Lenken der Tröpfchen in den Druckluftstrom, so dass die Tröpfchen auf eine Oberfläche eines Substrats gelenkt werden und die Oberfläche **dadurch** mit der Antikoagulans-Lösung beschichtet wird.

2. Verfahren gemäß Anspruch 1, das weiterhin das Trocknen der Antikoagulans-Lösung auf der Oberfläche des Substrats umfasst, indem man warme Luft über die Oberfläche strömen lässt, so dass Wasser aus der Antikoagulans-Lösung verdunstet und eine Beschichtung aus festem Antikoagulans darauf zurückbleibt.

3. Verfahren gemäß Anspruch 2, wobei die warme Luft wenigstens etwa 40 °C warm ist; und
wobei die warme Luft weiterhin nicht mehr als etwa 60 °C warm ist.

4. Verfahren gemäß Anspruch 2, wobei man die warme Luft wenigstens etwa 5 Sekunden lang über die Oberfläche strömen lässt; und
wobei man die warme Luft weiterhin nicht länger als etwa 60 Sekunden lang über die Oberfläche strömen lässt.

5. Verfahren gemäß Anspruch 2, wobei man die warme Luft mit einer Geschwindigkeit von wenigstens etwa 5 Metern pro Sekunde über die Oberfläche strömen lässt; und
wobei man die warme Luft weiterhin mit einer Geschwindigkeit von nicht mehr als etwa 15 Metern pro Sekunde über die Oberfläche strömen lässt.

6. Verfahren gemäß Anspruch 1, wobei die Antikoagulans-Lösung Ethylendiamintetraessigsäure oder Heparin enthält.

7. Verfahren gemäß Anspruch 1, wobei die Antikoagulans-Lösung ein mit Calcium ausgeglichenes Lithiumheparin mit einer Heparinkonzentration von wenigstens etwa 3000 IE pro Milliliter ist; und
wobei die Antikoagulans-Lösung weiterhin eine Heparinkonzentration von nicht mehr als etwa 10 000 IE pro Milliliter hat.

8. Verfahren gemäß Anspruch 1, wobei die Antikoagulans-Lösung ein mit Calcium ausgeglichenes Lithiumheparin mit einer Heparinkonzentration von wenigstens etwa 6500 IE pro Milliliter ist; und
wobei die Antikoagulans-Lösung weiterhin eine Heparinkonzentration von nicht mehr als etwa 7500 IE pro Milliliter hat.

9. Verfahren gemäß Anspruch 1, wobei die Oberfläche ein inneres Teil einer Spritze ist.

10. Verfahren gemäß Anspruch 9, wobei das Zerstäuben das Zerstäuben von wenigstens etwa 1 Mikroliter der Antikoagulans-Lösung umfasst; und
wobei das Zerstäuben weiterhin das Zerstäuben von nicht mehr als etwa 20 Mikroliter der Antikoagulans-Lösung umfasst.

11. Verfahren gemäß Anspruch 1, das weiterhin den Schritt des Hydrophobierens der Oberfläche des Substrats vor dem Zerstäuben der Antikoagulans-Lösung umfasst.

12. Verfahren gemäß Anspruch 11, wobei der Schritt das Beschichten der Oberfläche mit einem Silikonöl umfasst.

13. Verfahren zur Beschichtung eines inneren Teils einer Spritze mit einem Antikoagulans, umfassend:
Bereitstellen einer konzentrierten wässrigen Antikoagulans-Lösung;
Bereitstellen eines Druckluftstroms;
Bereitstellen von wenigstens zwei Sprühdüsen mit jeweils einer ersten Kammer in Fluid-Verbindung mit dem Druckluftstrom und jeweils einer zweiten Kammer in Fluid-Verbindung mit der Antikoagulans-Lösung;
Zerstäuben von etwa 1 bis etwa 20 Mikroliter der Antikoagulans-Lösung zu feinen Nebeltröpfchen, während die Antikoagulans-Lösung aus der zweiten Kammer einer der Sprühdüsen austritt;
Lenken der Tröpfchen in den Druckluftstrom, der aus der einen Sprühdüse austritt, so dass die Tröpfchen auf einen inneren Teil einer Spritze gelenkt werden und dieser Teil **dadurch** mit der Antikoagulans-Lösung beschichtet wird;
Zerstäuben von etwa 1 bis etwa 20 Mikroliter der Antikoagulans-Lösung zu feinen Nebeltröpfchen, während die Antikoagulans-Lösung aus der zweiten Kammer der anderen Sprühdüse austritt;
Lenken der Tröpfchen in den Druckluftstrom, der aus der anderen Sprühdüse austritt, so dass die Tröpfchen auf den Teil der Spritze gelenkt werden und dieser Teil **dadurch** mit der Antikoagulans-Lösung beschichtet wird; und
Trocknen der Antikoagulans-Lösung auf der Oberfläche des Substrats, so dass Wasser aus der Antikoagulans-Lösung verdunstet und eine physikalische Beschichtung aus festem Antikoagulans darauf zurückbleibt.

14. Verfahren gemäß Anspruch 13, wobei das Trocknen weiterhin das Strömenlassen von warmer Luft über die Oberfläche umfasst.

15. Verfahren gemäß Anspruch 13, wobei die Antikoagulans-Lösung ein mit Calcium ausgeglichenes Lithiumheparin mit einer Heparinkonzentration von wenigstens etwa 3000 IE pro Milliliter ist; und
wobei die Antikoagulans-Lösung weiterhin eine Heparinkonzentration von nicht mehr als etwa 10 000 IE pro Milliliter hat.

16. Verfahren gemäß Anspruch 13, wobei die Antikoagulans-Lösung ein mit Calcium ausgeglichenes Lithiumheparin mit einer Heparinkonzentration von wenigstens etwa 6500 IE pro Milliliter ist; und
wobei die Antikoagulans-Lösung weiterhin eine Heparinkonzentration von nicht mehr als etwa 7500 IE pro Milliliter hat.

17. Verfahren gemäß Anspruch 13, das weiterhin das Beschichten der Oberfläche mit einem Silikonöl vor dem Zerstäuben der Antikoagulans-Lösung umfasst.

18. Spritze zur Entnahme einer Blutprobe, umfassend:
einen inneren Teil, der eine physikalische Beschichtung aus luftgetrocknetem, mit Calcium ausgeglichenem Lithiumheparin aufweist, wobei die Beschichtung abgeschieden wurde, indem man 1 bis 20 Mikroliter einer konzentrierten wässrigen Lösung von mit Calcium ausgeglichenem Lithiumheparin zerstäubt.

19. Spritze gemäß Anspruch 18, wobei die konzentrierte wässrige Lösung des mit Calcium ausgeglichenen Lithiumheparins wenigstens etwa 3000 IE Heparin pro Milliliter enthält; und
wobei die konzentrierte wässrige Lösung des mit Calcium ausgeglichenen Lithiumheparins weiterhin nicht mehr als etwa 10 000 IE Heparin pro Milliliter enthält.

20. Spritze gemäß Anspruch 19, wobei die Spritze eine nominelle Größe von etwa 1 cm³ bis etwa 5 cm³ hat.

21. Spritze gemäß Anspruch 18, wobei die konzentrierte wässrige Lösung des mit Calcium ausgeglichenen Lithiumheparins wenigstens etwa 6500 IE Heparin pro Milliliter enthält; und
wobei die konzentrierte wässrige Lösung des mit Calcium ausgeglichenen Lithiumheparins weiterhin nicht mehr als etwa 7500 IE Heparin pro Milliliter enthält.

22. Spritze gemäß Anspruch 21, wobei die Spritze eine nominelle Größe von etwa 1 cm³ bis etwa 5 cm³ hat.

## Revendications

1. Procédé pour revêtir un substrat avec un anticoagulant, comprenant les étapes consistant à :
fournir une solution aqueuse concentrée d'anticoagulant ;
fournir un flux d'air pressurisé ;
fournir une buse ayant une première chambre en communication de fluide avec ledit flux d'air pressurisé et ayant une seconde chambre en communication de fluide avec ladite solution d'anticoagulant ;
atomiser ladite solution d'anticoagulant en fines gouttelettes de brouillard lorsque ladite solution d'anticoagulant sort de ladite seconde chambre ; et
diriger lesdites gouttelettes dans ledit flux d'air pressurisé pour diriger lesdites gouttelettes vers une surface d'un substrat pour revêtir ladite surface avec ladite solution d'anticoagulant.

2. Procédé selon la revendication 1 comprenant en outre l'étape consistant à sécher ladite solution d'anticoagulant sur ladite surface dudit substrat en forçant de l'air chaud sur ladite surface pour évaporer l'eau de ladite solution d'anticoagulant pour y laisser un revêtement d'anticoagulant solide.

3. Procédé selon la revendication 2 dans lequel ledit air chaud est à au moins environ 40 °C ; et dans lequel en outre ledit air chaud n'est pas à plus d'environ 60 °C.

4. Procédé selon la revendication 2 dans lequel ledit air chaud est forcé sur ladite surface depuis au moins environ 5 secondes ; et
dans lequel en outre ledit air chaud est forcé sur ladite surface depuis pas plus d'environ 60 secondes.

5. Procédé selon la revendication 2 dans lequel ledit air chaud est forcé sur ladite surface à une vitesse d'au moins environ 5 mètres par seconde ; et
dans lequel en outre ledit air chaud est forcé sur ladite surface à une vitesse qui n'est pas supérieure à environ 15 mètres par seconde.

6. Procédé selon la revendication 1 dans lequel ladite solution d'anticoagulant comprend de l'acide éthylène diamine tétra acétique ou de l'héparine.

7. Procédé selon la revendication 1 dans lequel ladite solution d'anticoagulant est une héparine de lithium équilibrée en calcium ayant une concentration en héparine d'au moins environ 3 000 UI par millilitre ;
et dans lequel en outre ladite solution d'anticoagulant a une concentration en héparine ne dépassant pas environ 10 000 UI par millilitre.

8. Procédé selon la revendication 1 dans lequel ladite solution d'anticoagulant est une héparine de lithium équilibrée en calcium ayant une concentration en héparine d'au moins environ 6 500 UI par millilitre ;
et dans lequel en outre ladite solution d'anticoagulant a une concentration en héparine ne dépassant pas environ 7 500 UI par millilitre.

9. Procédé selon la revendication 1 dans lequel ladite surface est la partie intérieure d'une seringue.

10. Procédé selon la revendication 9 dans lequel ladite atomisation inclut l'atomisation d'au moins environ 1 microlitre de ladite solution d'anticoagulant ; et
dans lequel en outre ladite atomisation inclut l'atomisation de pas plus d'environ 20 microlitres de ladite solution d'anticoagulant.

11. Procédé selon la revendication 1 comprenant en outre l'étape consistant à fournir une hydrophobicité à ladite surface dudit substrat avant atomisation de ladite solution d'anticoagulant.

12. Etape de la revendication 11 dans laquelle ladite étape inclut le revêtement de ladite surface avec une huile de silicone.

13. Procédé pour revêtir la partie intérieure d'une seringue avec un anticoagulant comprenant les étapes consistant à :
fournir une solution aqueuse concentrée d'anticoagulant ;
fournir un flux d'air pressurisé ;
fournir au moins deux buses de pulvérisation ayant chacune une première chambre en communication de fluide avec ledit flux d'air pressurisé et ayant chacune une seconde chambre en communication de fluide avec ladite solution d'anticoagulant ;
atomiser entre environ 1 et environ 20 microlitres de ladite solution d'anticoagulant en fines gouttelettes de brouillard lorsque ladite solution d'anticoagulant sort de ladite seconde chambré de l'une desdites buses de pulvérisation ;
diriger lesdites gouttelettes dans ledit flux d'air pressurisé sortant de ladite une buse de pulvérisation pour diriger lesdites gouttelettes vers une partie intérieure d'une seringue pour revêtir ladite partie avec ladite solution d'anticoagulant ;
atomiser entre environ 1 et environ 20 microlitres de ladite solution d'anticoagulant en fines gouttelettes de brouillard lorsque ladite solution d'anticoagulant sort de ladite seconde chambre de l'autre desdites buses de pulvérisation ;
diriger lesdites gouttelettes dans ledit flux d'air pressurisé sortant de ladite autre buse de pulvérisation pour diriger lesdites gouttelettes vers ladite partie de ladite seringue pour revêtir ladite partie avec ladite solution d'anticoagulant ; et
sécher ladite solution d'anticoagulant sur ladite surface dudit substrat pour évaporer l'eau de ladite solution d'anticoagulant pour y laisser un revêtement physique d'anticoagulant solide.

14. Procédé selon la revendication 13 dans lequel ledit séchage inclut en outre l'étape consistant à forcer de l'air chaud sur ladite surface.

15. Procédé selon la revendication 13 dans lequel ladite solution d'anticoagulant est une héparine de lithium équilibrée en calcium ayant une concentration en héparine d'au moins environ 3 000 UI par millilitre ;
et dans lequel en outre ladite solution d'anticoagulant a une concentration en héparine ne dépassant pas environ 10 000 UI par millilitre.

16. Procédé selon la revendication 13 dans lequel ladite solution d'anticoagulant est une héparine de lithium équilibrée en calcium ayant une concentration en héparine d'au moins environ 6 500 UI par millilitre ;
et dans lequel en outre ladite solution d'anticoagulant a une concentration en héparine ne dépassant pas environ 7 500 UI par millilitre.

17. Procédé selon la revendication 13 comprenant en outre le revêtement de ladite surface avec une huile de silicone avant atomisation de ladite solution d'anticoagulant.

18. Seringue pour prélever un échantillon de sang comprenant :
une partie intérieure ayant un revêtement physique d'héparine de lithium équilibrée en calcium séchée à l'air ; ledit revêtement étant déposé par atomisation de 1 à 20 microlitres d'une solution aqueuse concentrée d'héparine de lithium équilibrée en calcium.

19. Seringue selon la revendication 18 dans laquelle ladite solution aqueuse concentrée d'héparine de lithium équilibrée en calcium contient au moins environ 3 000 UI d'héparine par millilitre ; et
dans laquelle en outre ladite solution aqueuse concentrée d'héparine de lithium équilibrée en calcium ne contient pas plus de 10 000 UI d'héparine par millilitre.

20. Seringue selon la revendication 19 dans laquelle ladite seringue a une taille nominale d'environ 1 cm³ à environ 5 cm³.

21. Seringue selon la revendication 18 dans laquelle ladite solution aqueuse concentrée d'héparine de lithium équilibrée en calcium contient au mois environ 6 500 UI d'héparine par millilitre ; et dans laquelle en outre ladite solution aqueuse concentrée d'héparine de lithium équilibrée en calcium ne contient pas plus de 7 500 UI d'héparine par millilitre.

22. Seringue selon la revendication 21 dans laquelle ladite seringue a une taille nominale d'environ 1 cm³ à environ 5 cm³.
